# EUROPEAN PATENT SPECIFICATION

(11) **EP 4 179 954 B1**
(45) Date of publication and mention of the grant of the patent: **14.08.2024**
(21) Application number: 20959728.5
(22) Date of filing: 27.10.2020
(51) Int. Cl.: A61B 1/045, A61B 3/13

(54) **SURGERY ASSISTING DEVICE**
OPERATIONSUNTERSTÜTZUNGSVORRICHTUNG
DISPOSITIF D'AIDE À LA CHIRURGIE

(43) Date of publication of application: 17.05.2023
(73) Proprietor: RIVERFIELD Inc., 1070052 Tokyo (JP)
(72) Inventor: MORIKAWA, Atsushi, Tokyo 160-0017 (JP); TADANO, Kotaro, Tokyo 160-0017 (JP)
(74) Representative: adares Patent- und Rechtsanwälte Reininger & Partner GmbB
(86) International application number: PCT/JP2020/040231
(87) International publication number: WO 2022/091210

(56) References cited:
- EP-A1- 3 437 593
- WO-A1-2017/169823
- WO-A1-2018/088107
- WO-A1-2018/088107
- JP-A- 2008 262 555
- JP-A- H11 309
- JP-A- H11 309
- US-A1- 2005 020 883
- US-A1- 2020 154 987

## Description

### Technical Field

The present invention relates to a technical field of a surgery assisting device having a function of holding an endoscope.

### Background Art

Vitreous body surgery is known which restores a retina to a normal state by sucking and removing a vitreous body within an eyeball, for example, for treatment of maculopathy, retinal detachment, or the like.

In ordinary vitreous body surgery, an operator (surgeon) observes the inside of the eyeball through the pupil of a subject (patient) by a surgical microscope or the like. There is a limit to a range in which the inside of the eyeball can be observed through the pupil. In order to bring the part that cannot be observed into a viewable range, the eyeball needs to be pressed from the outside. Such pressing may cause a pain during the surgery or inflammation after the surgery.

Accordingly, a method of using an endoscope for vitreous body surgery as illustrated in PTL 1 has been proposed.

When the endoscope is inserted into the eyeball of the subject, an image of the inside of the eyeball is displayed by display means such as a monitor. The operator can easily observe a part normally unable to be viewed from the pupil by moving the endoscope within the eyeball.

The vitreous body surgery using such an endoscope obviates a need for pressing the eyeball in observing the inside of the eyeball. A burden on the eyeball of the subject can therefore be reduced.

JP H11 309 A relates to an image display device for displaying an image taken by an endoscope and a three-dimensional image of an organ of a subject corresponding to the three-dimensional position and line-of-sight direction of the endoscope.

### Citation List

### Patent Literature

PTL 1: JP 2005-304633 A

### Summary of Invention

### Technical Problem

The vitreous body surgery is performed by inserting treatment instruments such as a vitreous body cutter, forceps, and an injector of a perfusate or the like into the eyeball. The operator performs surgery on the inside of the eyeball while checking an endoscope captured image based on imaging by the endoscope on a monitor.

In the surgery using such an endoscope, it is not easy for the operator to estimate an inserted state of the endoscope with respect to the eyeball of the subject and grasp which position within the eyeball is captured in the endoscope captured image displayed on the monitor.

It is accordingly an object of the present invention to make display that makes it possible to spatially grasp the inserted state of the endoscope with respect to the subject. Solution to Problem

A surgery assisting device according to the present invention includes an arm unit including a holder for holding an endoscope and configured to adjust a position of the endoscope in a state in which the holder holds the endoscope, an image generating section configured to generate an endoscope viewpoint map image that is an image of a three-dimensional model of a subject from a viewpoint of the endoscope held by the holder, a display control section configured to perform display control of the endoscope viewpoint map image and an endoscope captured image that is obtained by the endoscope, and an operating unit for rotating the endoscope captured image. The displayed endoscope captured image is rotated according to operation of the operating unit without a positional displacement of the endoscope being caused, and the endoscope viewpoint map image is rotated in conjunction with the rotation of the endoscope captured image.

The endoscope viewpoint map image is consequently displayed which corresponds to the state of the endoscope captured image after being rotated and indicates positional relation between the endoscope and the subject.

In the surgery assisting device described above, the rotation of the endoscope viewpoint map image may be performed in a state in which a position of an image illustrating the endoscope is fixed.

Consequently, the endoscope viewpoint map image is displayed in a state in which the image illustrating the endoscope always appears irrespective of the rotational state of the endoscope captured image.

In the surgery assisting device described above, the display control section may display the endoscope viewpoint map image and the endoscope captured image within the same screen.

Consequently, in performing surgery while visually checking the endoscope captured image, it is possible to check the endoscope viewpoint map image without moving a line of sight to another monitor.

In the surgery assisting device described above, the display control section may display an ocular map image indicating the position of the endoscope on a three-dimensional ocular model and the endoscope viewpoint map image within the same screen.

Consequently, it is possible to check the ocular map image without moving the line of sight to another monitor when visually checking the endoscope viewpoint map image.

### Advantageous Effect of Invention

According to the present invention, it is possible to spatially grasp the inserted state of the endoscope with respect to the subject.

### Brief Description of Drawings

FIG. 1 is a diagram schematically illustrating an example of a configuration of a surgery system in an embodiment of the present invention.
FIG. 2 is a diagram schematically illustrating a sectional configuration of an eyeball of a subject in the present embodiment.
FIG. 3 is a diagram schematically illustrating an example of a configuration of a surgery assisting device in the present embodiment.
FIG. 4 is a diagram schematically illustrating an example of a configuration of an arm distal end section of an endoscope holding device in the present embodiment.
FIG. 5 is a diagram illustrating an example of a display image displayed on a monitor in the present embodiment.
FIG. 6 is a block diagram illustrating an example of a configuration of the surgery assisting device in the present embodiment.
FIG. 7 is a flowchart illustrating an example of processing performed by a computation and control unit of the surgery assisting device in the present embodiment.
FIG. 8 is a diagram illustrating an outline of a procedure for determining positional relation between the eyeball and an endoscope in the present embodiment.
FIG. 9 is a flowchart illustrating an example of processing performed by the computation and control unit of the surgery assisting device in the present embodiment.
FIG. 10 is a flowchart illustrating an example of endoscope viewpoint map image generation processing performed by the computation and control unit in the present embodiment.
FIG. 11 is a diagram illustrating an example of an endoscope captured image and an endoscope viewpoint map image in the display image displayed on the monitor in the present embodiment.

### Description of Embodiment

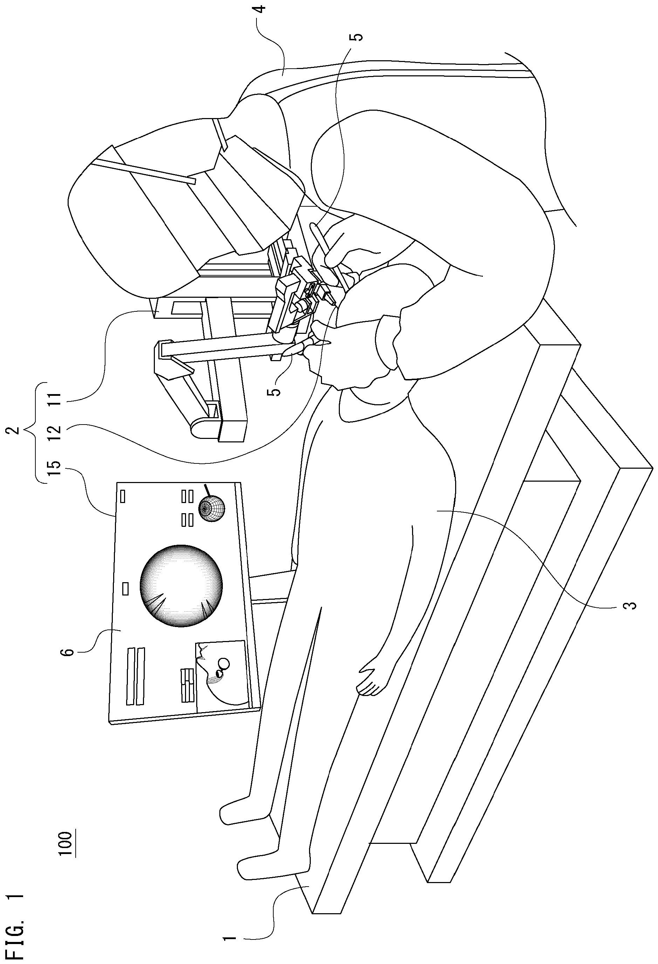

An embodiment of the present invention will be described with reference to FIGS. 1 to 11. The drawings extract and illustrate principal parts and peripheral configurations thereof recognized to be necessary for description. In addition, the drawings are schematic, and the dimensions, ratios, and the like of respective structures included in the drawings are mere examples. Hence, various changes can be made according to design or the like without departing from technical ideas of the present invention. In addition, configurations described once may be subsequently identified by the same reference numerals, and description thereof may be omitted.

The embodiment will hereinafter be described in the following order.

### <1. Configuration of Surgery System>

### <2. Functional Configuration of Computation and Control Unit>

### <3. Example of Processing of Embodiment>

### <4. Summary>

### <1. Configuration of Surgery System>

A configuration of a surgery system 100 in ocular surgery will be described.

FIG. 1 schematically illustrates an example of the configuration of the surgery system 100.

The surgery system 100 includes an operating table 1 and a surgery assisting device 2.

The operating table 1 and the surgery assisting device 2 are installed in an operating room.

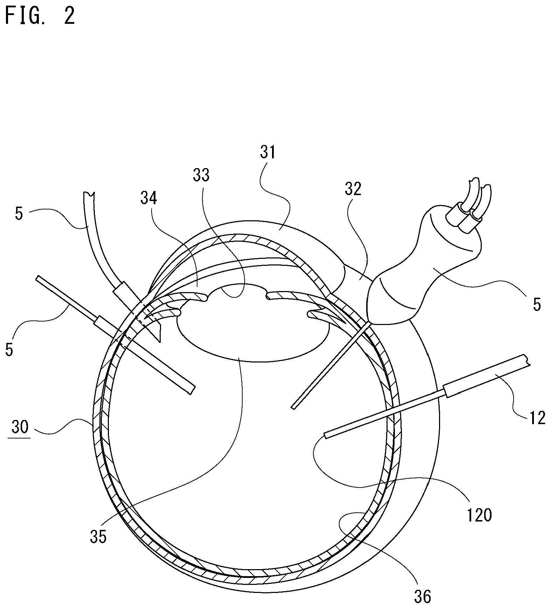

A subject (patient) 3 is laid down on his or her back on the operating table 1. An operator (surgeon) 4 is positioned on the head side of the subject 3, and performs surgery within an eyeball 30 (see FIG. 2) of the subject 3 by using various kinds of treatment instruments 5. Used as the treatment instruments 5 are, for example, a vitreous body cutter, forceps, an injector of a perfusate or the like, and the like.

FIG. 2 schematically illustrates a sectional configuration of the eyeball 30. The surface of the eyeball 30 is covered by a cornea 31 and a conjunctiva 32. An iris 34 in which a pupil 33 is formed is present in the rear of the cornea 31. A crystalline lens 35 is present in the rear of the iris 34. In addition, a retina 36 is present on the whole surface of an ocular fundus within the eyeball 30.

The operator 4, for example, inserts the treatment instruments 5 through the conjunctiva 32, and performs surgery within the eyeball 30.

The surgery assisting device 2 assists in the surgery on the eyeball 30 by the operator 4.

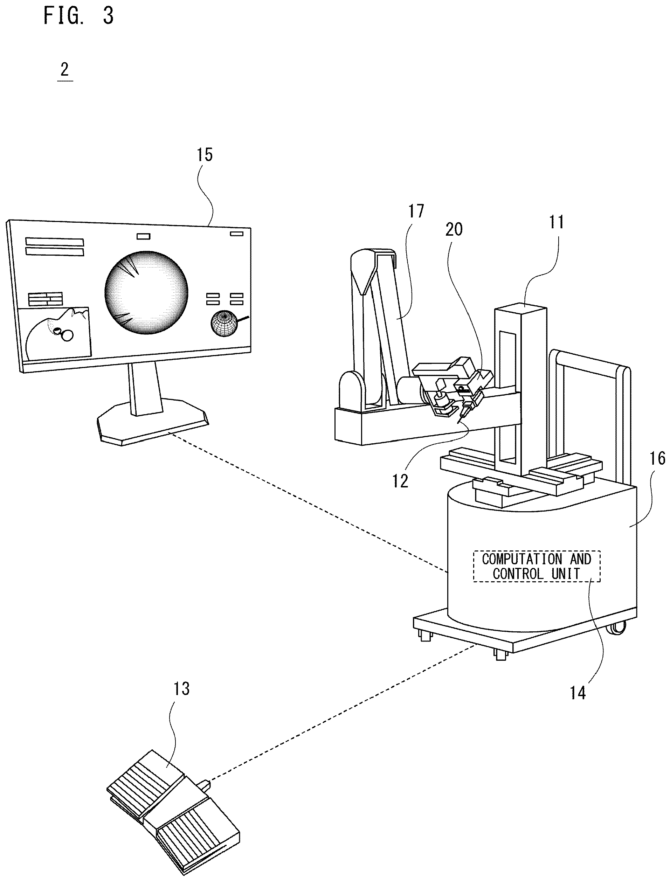

FIG. 3 schematically illustrates an example of a configuration of the surgery assisting device 2.

The surgery assisting device 2 includes an endoscope holding device 11, an endoscope 12, an operating unit 13, a computation and control unit 14, and a monitor 15.

The endoscope holding device 11 includes a base unit 16 and an arm unit 17.

The base unit 16 is mounted on the floor of the operating room or the like. The arm unit 17 is attached to the base unit 16. The arm unit 17 is pivotally supported by the base unit 16 in a rotatable manner.

The arm unit 17 includes one or a plurality of joint sections and rotary sections, and is formed as a mechanism that can move an arm distal end section 20 to a given position.

A configuration of the arm distal end section 20 will be described in the following.

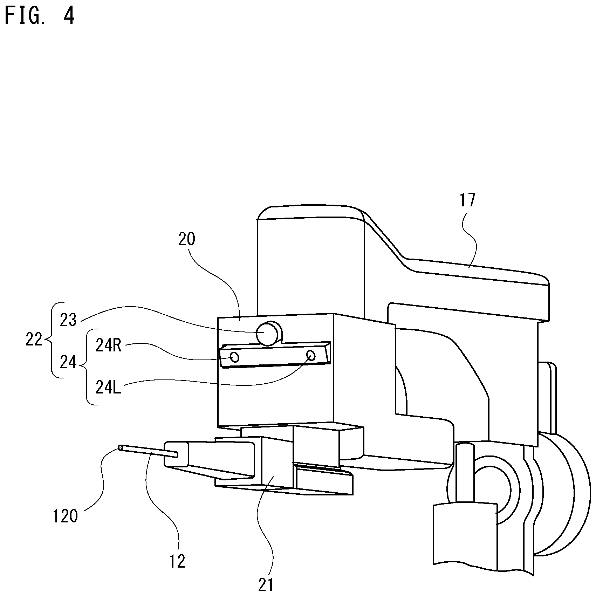

FIG. 4 schematically illustrates an example of the configuration of the arm distal end section 20.

The arm distal end section 20 includes a holder 21 for holding the endoscope 12 and a measuring unit 22 used to measure a distance to the cornea 31 of the subject 3.

The holder 21 is formed as a mechanism that allows the endoscope 12 to be attached to and detached from the holder 21. The endoscope 12 is fixed to the holder 21 by fitting the endoscope 12 into the holder 21. The endoscope 12 can be freely moved to a given position by operating the arm unit 17 in a state in which the endoscope 12 is fixed to the holder 21.

When the holder 21 holds the endoscope 12 inserted in the eyeball 30 of the subject 3, the operator 4 does not need to hold the endoscope 12 with a hand. Hence, the operator 4 can perform surgery on the eyeball 30 with both hands.

The measuring unit 22 includes an irradiating unit 23 and an imaging unit 24.

The irradiating unit 23 includes an LED (Light Emitting Diode), for example. The irradiating unit 23 outputs light that irradiates the eyeball 30 of the subject 3.

The imaging unit 24 includes imaging units 24L and 24R to be able to perform distance measurement by what is called a stereo method. The imaging units 24L and 24R are, for example, arranged at a predetermined interval from each other in the vicinity of an upper portion of the holder 21. Optical axes of the imaging units 24L and 24R are parallel with each other, and respective focal lengths of the imaging units 24L and 24R are the same value. In addition, frame periods thereof are in synchronism with each other, and frame rates thereof coincide with each other.

Captured image signals obtained by respective imaging elements of the imaging units 24L and 24R are each subjected to A/D (Analog/Digital) conversion, and are thereby converted into digital image signals (captured image data) indicating luminance values based on a predetermined gray scale in pixel units.

Distances from the imaging units 24L and 24R to the cornea 31 of the subject 3 can be measured on the basis of the captured image signals obtained by the respective imaging elements of the imaging units 24L and 24R in a state in which the irradiating unit 23 irradiates the eyeball 30.

In the measuring unit 22, relative positional relation between the irradiating unit 23 and the imaging units 24L and 24R is fixed. In addition, relative positional relation between the imaging units 24L and 24R and the above-described holder 21 is fixed. Hence, relative positional relation of the irradiating unit 23 and the imaging units 24L and 24R to the endoscope 12 is fixed by fixing the endoscope 12 to the holder 21.

Returning to FIG. 3, the endoscope 12 of the surgery assisting device 2 is inserted into the eyeball 30 in a state in which the endoscope 12 is fixed to the holder 21 (see FIG. 2). A state within the eyeball 30 is obtained by the inserted endoscope 12. Captured image signals obtained by the imaging element of the endoscope 12 are each subjected to A/D conversion, and are thereby converted into digital image signals (captured image data) indicating luminance values based on a predetermined gray scale in pixel units.

A captured image based on the captured image data from the endoscope 12 is displayed on the liquid crystal of the monitor 15.

The operating unit 13 comprehensively represents operating equipment used to perform an operation of the arm unit 17, a rotational operation of the captured image, which is displayed on the monitor 15, based on imaging by the endoscope 12, and the like. The operating unit 13 may be a foot pedal, or may be a manually operated remote operating device (remote controller) or the like. FIG. 3 illustrates a foot pedal as an example. However, as described above, the operating unit 13 is not limited to this.

Details of a method for rotational operation of the captured image obtained by the endoscope 12 according to an operation of the operating unit 13 will be described later.

The computation and control unit 14 performs various kinds of processing necessary to implement the present embodiment, such as control of operation of the arm unit 17, processing of generating various kinds of images to be displayed on the monitor 15, and processing of controlling display on the monitor 15.

The computation and control unit 14, for example, includes a microcomputer including a CPU (Central Processing Unit), a ROM (Read Only Memory), a RAM (Random Access Memory), and the like. The computation and control unit 14 is implemented by one or a plurality of microcomputers.

The computation and control unit 14 is, for example, included in the base unit 16 of the endoscope holding device 11. Incidentally, the computation and control device 14 may be included in another external apparatus.

The monitor 15 displays a display image 6 on the liquid crystal under display control from the computation and control unit 14.

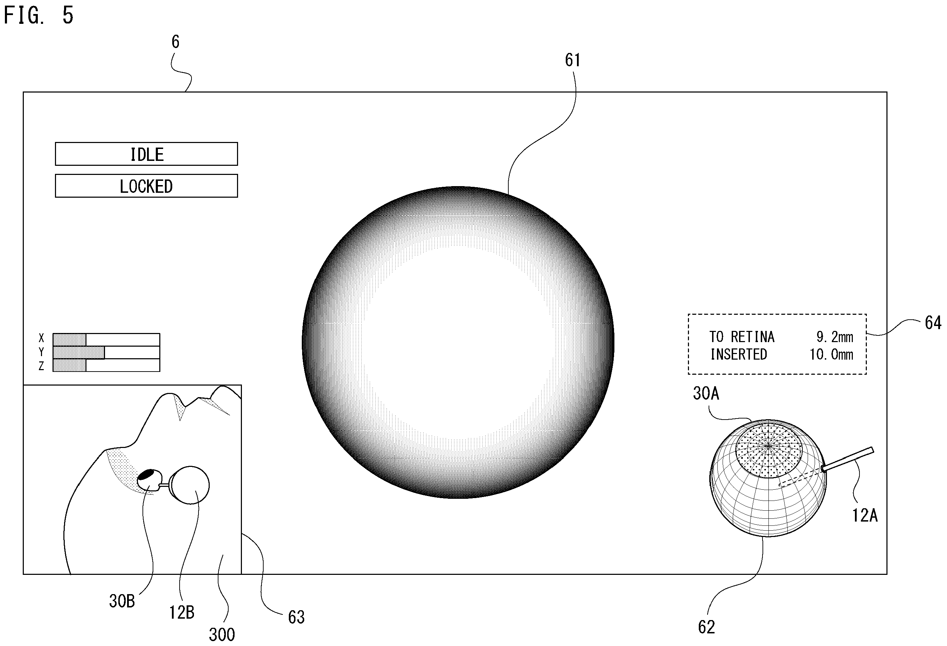

FIG. 5 illustrates an example of the display image 6 displayed on the monitor 15.

The display image 6, for example, including an endoscope captured image 61, an ocular map image 62, an endoscope viewpoint map image 63, an insertion length presenting image 64, and the like is displayed on the monitor 15. The display image 6 also includes images related to various kinds of information as required.

The endoscope captured image 61 is the captured image based on the captured image data from the endoscope 12. A state inside the eyeball 30, which is obtained by the endoscope 12, for example, is displayed as the endoscope captured image 61. The endoscope captured image 61 can be rotated by an operation using the operating unit 13. Details of a method for rotating the endoscope captured image 61 will be described later.

The ocular map image 62 illustrates positional relation between the eyeball 30 and the endoscope 12.

The ocular map image 62 displays the eyeball 30 by a three-dimensional ocular model image 30A. In addition, the position of the endoscope 12 with respect to the eyeball 30 is displayed by an endoscope model image 12A.

The endoscope viewpoint map image 63 displays an endoscope model image 12B illustrating the endoscope 12 and a three-dimensional model image 300 of the subject 3 from the viewpoint of the endoscope 12. The three-dimensional model image 300 displays an ocular model image 30B.

The endoscope viewpoint map image 63 is rotated in conjunction with rotation of the endoscope captured image 61. At this time, in the endoscope viewpoint map image 63, the three-dimensional model image 300 is rotated in a state in which the position of the endoscope model image 12B illustrating the endoscope 12 is fixed. Details of a method for rotating the endoscope viewpoint map image 63 will be described later.

The insertion length presenting image 64 displays the numerical value of an insertion length of the endoscope 12 with respect to the eyeball 30 and the numerical value of a distance from an endoscope distal end portion 120 to the retina 36.

The operator 4 performs surgery on the eyeball 30 while checking the display image 6 displayed on the monitor 15.

### <2. Functional Configuration of Computation and Control Unit>

A functional configuration of the computation and control unit 14 in the surgery assisting device 2 will be described.

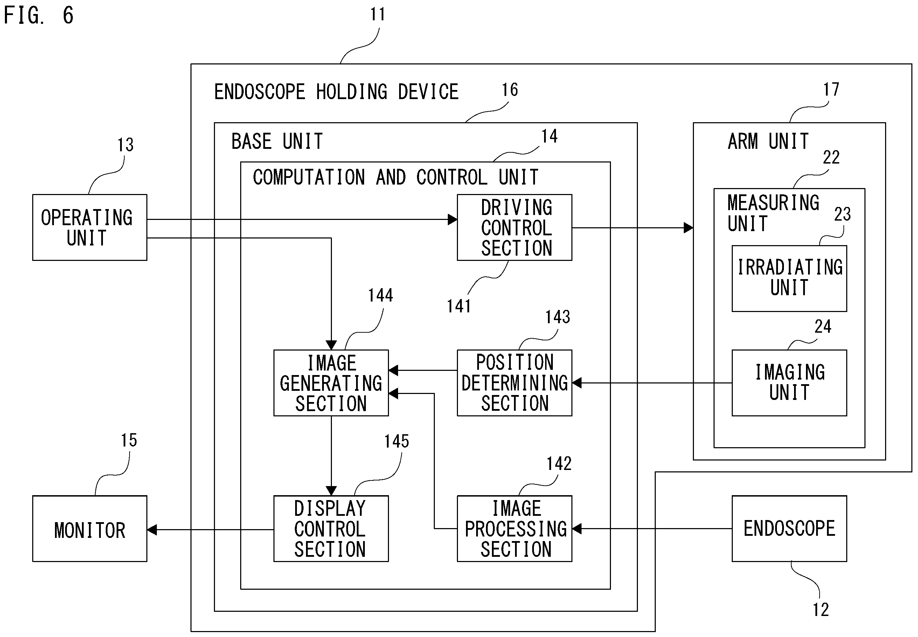

FIG. 6 illustrates, as a block diagram, an example of a configuration of the surgery assisting device 2.

The computation and control unit 14 includes a driving control section 141, an image processing section 142, a position determining section 143, an image generating section 144, and a display control section 145.

The driving control section 141 performs operation control on the joint section(s) and the rotary section(s) of the arm unit 17 of the endoscope holding device 11 on the basis of an operation signal input from the operating unit 13, for example. The driving control section 141 can move the position of the endoscope 12 fixed to the holder 21 of the arm distal end section 20 by performing operation control on the arm unit 17.

In addition, the driving control section 141 performs output control on the irradiating unit 23 and imaging control on the imaging unit 24.

The image processing section 142 subjects the image signal based on imaging by the endoscope 12 to various kinds of signal processing such as luminance signal processing, color processing, resolution conversion processing, and codec processing. The image processing section 142 outputs the image signal resulting from the various kinds of signal processing to the image generating section 144.

The position determining section 143 computes an imaging distance, which is a distance from the imaging unit 24 to the cornea 31, on the basis of the captured image signals of the eyeball 30 from the respective imaging elements of the imaging units 24L and 24R, the captured image signals being input from the imaging unit 24.

In addition, the position determining section 143 computes relative positional relation between the eyeball 30 and the endoscope distal end portion 120 on the basis of the imaging distance.

The position determining section 143 outputs a determination result (relative positional relation between the eyeball 30 and the endoscope distal end portion 120) to the image generating section 144.

The image generating section 144 generates the display image 6 as illustrated in FIG. 5 by using various kinds of input information from the image processing section 142, the position determining section 143, the operating unit 13, and the like. Details of a method for generating various kinds of images constituting the display image 6 will be described later.

The image generating section 144 outputs an image signal of the generated display image 6 to the display control section 145.

The display control section 145 performs control that displays the display image 6 on the monitor 15 on the basis of the image signal input from the image generating section 144.

### <3. Example of Processing of Embodiment>

Description will be made of processing performed by the computation and control unit 14 of the surgery assisting device 2 in order to implement the present embodiment. Here, suppose, as an example, that the operator 4 is performing surgery while positioned on the head side of the subject 3 (see FIG. 1).

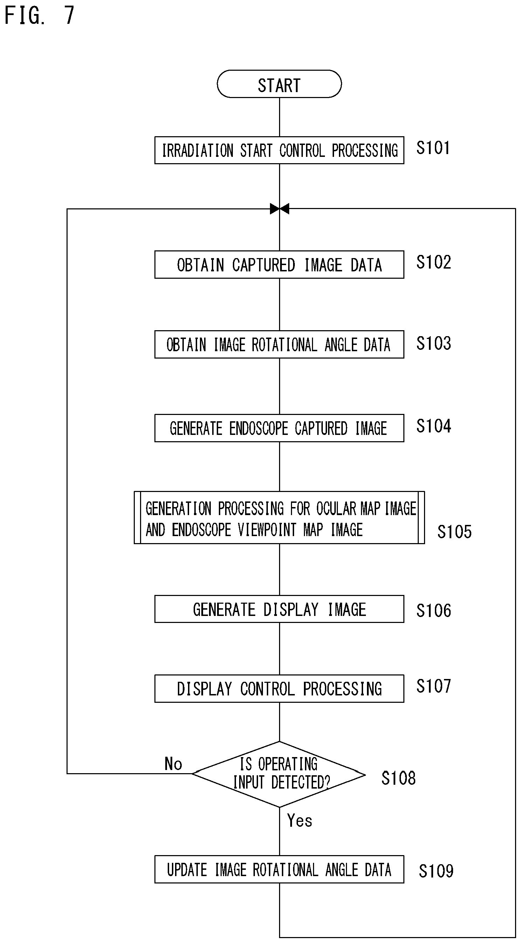

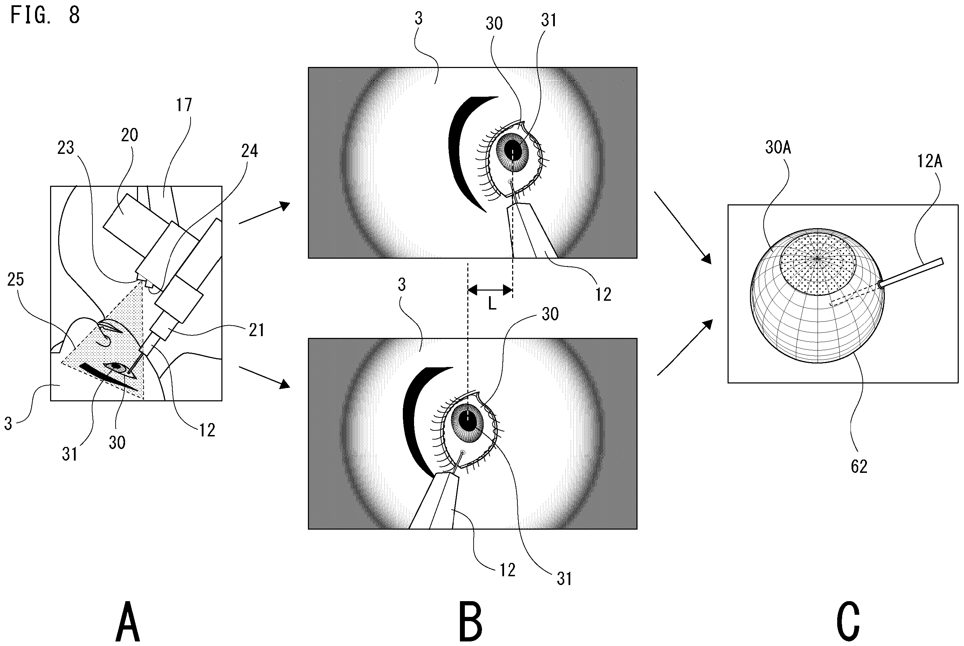

FIG. 7 is a flowchart illustrating an example of the processing performed by the computation and control unit 14. In addition, FIG. 8 illustrates an outline of a procedure for determining the positional relation between the eyeball 30 and the endoscope 12.

In step S101, the computation and control unit 14 performs irradiation start control processing.

In the irradiation start control processing, the computation and control unit 14 causes the irradiating unit 23 to output light 25 for irradiating the eyeball 30 as illustrated in FIG. 8A. Incidentally, in FIG. 8A, the light 25 output from the irradiating unit 23 is schematically indicated by a broken line.

The irradiation start control processing is processing necessary to determine the positional relation, which is used to generate image data of the endoscope viewpoint map image 63 to be described later, between the eyeball 30 and the endoscope 12.

Thereafter, the computation and control unit 14 repeatedly performs the processing from step S 102 on down in timing of each frame of the image.

In step S102, the computation and control unit 14 stores, in an internal memory, each piece of frame image data, which is the captured image data obtained by imaging the inside of the eyeball 30 by the endoscope 12.

In step S103, the computation and control unit 14 obtains image rotational angle data.

The image rotational angle data is information indicating a rotational direction and a rotational angle of the captured image data from the endoscope 12, which is used to perform rotation processing on the captured image data.

The image rotational angle data is updated as appropriate according to operating input from the operating unit 13. However, the image rotational angle data is not updated when an imaging direction of the endoscope 12 is shifted according to operation of the arm unit 17.

During a period from a start of the processing of FIG. 7 by the computation and control unit 14 to detection of an operating input by the operating unit 13 (the period will hereinafter be described also as an initial state), the image rotational angle data is a value indicating an angle of 0° in rotation processing on the endoscope captured image.

When the computation and control unit 14 detects an operating input from the operating unit 13, the computation and control unit 14 updates the image rotational angle data according to an operation amount of the operating input. The rotational direction is indicated by a positive or negative angle of the updated image rotational angle data, for example. Details of update processing on the image rotational angle data will be described later.

In the following step S104, the computation and control unit 14 generates image data of the endoscope captured image 61 on the basis of the captured image data from the endoscope 12 and the image rotational angle data.

In this case, the computation and control unit 14 at least generates the image data of the endoscope captured image 61 obtained by rotating the captured image data from the endoscope 12 on the basis of the direction and the angle based on the image rotational angle data.

In addition, in a case of the initial state, a rotational angle of 0° is indicated as image rotational angle data, and therefore the computation and control unit 14 generates the image data as the endoscope captured image 61 without rotating the captured image data from the endoscope 12.

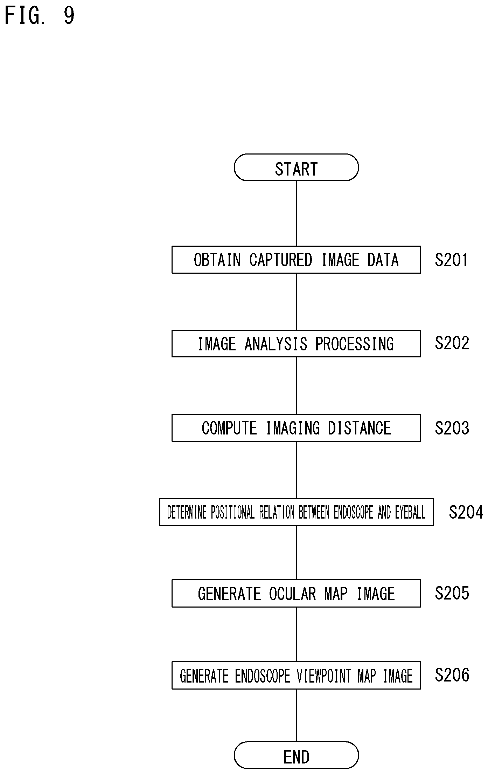

In step S105, the computation and control unit 14 performs image generation processing for the ocular map image 62 and the endoscope viewpoint map image 63. This image generation processing will be described with reference to FIG. 9. FIG. 9 is a flowchart illustrating an example of the image generation processing performed by the computation and control unit 14.

In step S201, the computation and control unit 14 stores, in the internal memory, respective pieces of frame image data, which is the captured image data obtained by imaging the eyeball 30 by the imaging units 24L and 24R.

In step S202, on the basis of two pieces of captured image data as each frame, the computation and control unit 14 performs various kinds of image analysis processing such, for example, as recognition of light spots of the light 25 from the irradiating unit 23, which appear on the eyeball 30 or the cornea 31 of the eyeball 30.

In step S203, for the pair of captured image data (stereo images) obtained by the imaging units 24L and 24R, the computation and control unit 14 computes the imaging distance, which is a distance from the imaging unit 24 to the cornea 31, by a principle of triangulation from an amount of offset L between the positions of the light spots appearing on the cornea 31, as illustrated in FIG. 8B.

The computation and control unit 14 determines positional relation between the imaging unit 24 and the eyeball 30 on the basis of the computed imaging distance.

At this time, ocular model data is used as data on the size of the eyeball 30.

The ocular model data is, for example, three-dimensional data assuming the eyeball size of an ordinary human. The eyeball size of humans does not differ greatly, though there are slight individual differences. Thus, the ocular model data is set in advance as ocular model data based on the standard eyeball size of a human.

The computation and control unit 14 can compute (determine) the positional relation between the imaging unit 24 and the eyeball 30 by using the imaging distance and the ocular model data.

Incidentally, it is also possible to measure the eyeball size of the eyeball 30 of the subject 3 in advance before the surgery and set the ocular model data by reflecting a result of the measurement.

In the following step S204, the computation and control unit 14 determines positional relation between the endoscope 12 and the eyeball 30 on the basis of the positional relation between the imaging unit 24 and the eyeball 30.

The relative positional relation of the endoscope 12 to the imaging unit 24 is fixed by fixing the endoscope 12 to the holder 21 of the arm distal end section 20. Therefore, in a state in which the endoscope 12 is fixed to the holder 21, the position of the endoscope 12 is defined naturally according to the position of the imaging unit 24.

In addition, a shape and size of the endoscope 12 up to the endoscope distal end portion 120 in an axial direction of the endoscope 12 fixed to the holder 21 is known. Therefore, when information regarding the shape and size of the endoscope 12 is set in advance, the computation and control unit 14 can compute the position of the endoscope distal end portion 120 from the defined position of the endoscope 12.

As a result of the above, the computation and control unit 14 can compute (determine) the positional relation of the endoscope 12 (endoscope distal end portion 120) to the eyeball 30 on the basis of the positional relation between the eyeball 30 and the imaging unit 24.

In the following step S205, the computation and control unit 14 generates image data of the ocular map image 62 indicating the determined positional relation between the eyeball 30 and the endoscope 12 (endoscope distal end portion 120) as positional relation between the three-dimensional ocular model image 30A and the endoscope model image 12A (see FIG. 8C).

Further, in step S206, the computation and control unit 14 generates image data of the endoscope viewpoint map image 63 as illustrated in FIG. 5. The endoscope viewpoint map image 63 includes the three-dimensional model image 300 illustrating a head portion of the subject 3 and the endoscope model image 12B illustrating the endoscope 12. The three-dimensional ocular model image 30B of the eyeball 30 is displayed in the three-dimensional model image 300.

Three-dimensional model data of a human, which is set in advance, is used for the three-dimensional model image 300 of the subject 3.

In addition, a value indicating the angle of the head portion of the subject 3 with respect to the endoscope 12 is set as head portion angle data in advance on an assumption that the subject 3 is laid down on his or her back on the operating table 1 and an installation angle of the endoscope holding device 11 with respect to the operating table 1 is defined.

The computation and control unit 14 generates image data of the three-dimensional model image 300 including the three-dimensional ocular model image 30B on the basis of the three-dimensional model data and the head portion angle data.

Then, the computation and control unit 14 generates the image data of the endoscope viewpoint map image 63 by synthesizing the three-dimensional model image 300 and the endoscope model image 12B on the basis of the positional relation between the eyeball 30 and the endoscope 12, which is determined in step S204.

The endoscope viewpoint map image 63 displays an image in which the head portion of the three-dimensional model image 300 is observed from the viewpoint of the endoscope 12.

In addition, the computation and control unit 14 generates the image data of the endoscope viewpoint map image 63 resulting from rotation processing on the basis of the image rotational angle data obtained in step S 103 in FIG. 7.

At this time, the computation and control unit 14 generates, as the endoscope viewpoint map image 63, image data obtained by rotating the three-dimensional model image 300 in a state in which the position of the endoscope model image 12B is fixed.

Incidentally, in a case of the initial state, a rotational angle of 0° is indicated as image rotational angle data, and therefore the computation and control unit 14 generates, as the endoscope viewpoint map image 63, image data obtained without rotating the endoscope viewpoint map image 63.

By ending the processing of step S206, the computation and control unit 14 completes the generation processing for the ocular map image 62 and the endoscope viewpoint map image 63 in S105 in FIG. 7.

The computation and control unit 14 next advances the processing to step S106.

In step S106, the computation and control unit 14 generates image data of the display image 6.

The computation and control unit 14 generates the display image 6 by synthesizing the endoscope captured image 61, the ocular map image 62, the endoscope viewpoint map image 63, the insertion length presenting image 64, and other necessary images.

Here, description will be made of an example of a method for generating image data of the insertion length presenting image 64.

The computation and control unit 14 computes information regarding the insertion length of the endoscope 12 in the eyeball 30 and information regarding the distance from the endoscope distal end portion 120 to the retina 36 of the subject 3 from the determined positional relation between the eyeball 30 and the endoscope 12 (endoscope distal end portion 120).

Then, the computation and control unit 14 generates the image data of the insertion length presenting image 64 on the basis of these pieces of information.

In the following step S107, the computation and control unit 14 performs display control for displaying the display image 6 on the liquid crystal of the monitor 15. The display image 6 as illustrated in FIG. 5 is thereby displayed within the same screen of the monitor 15.

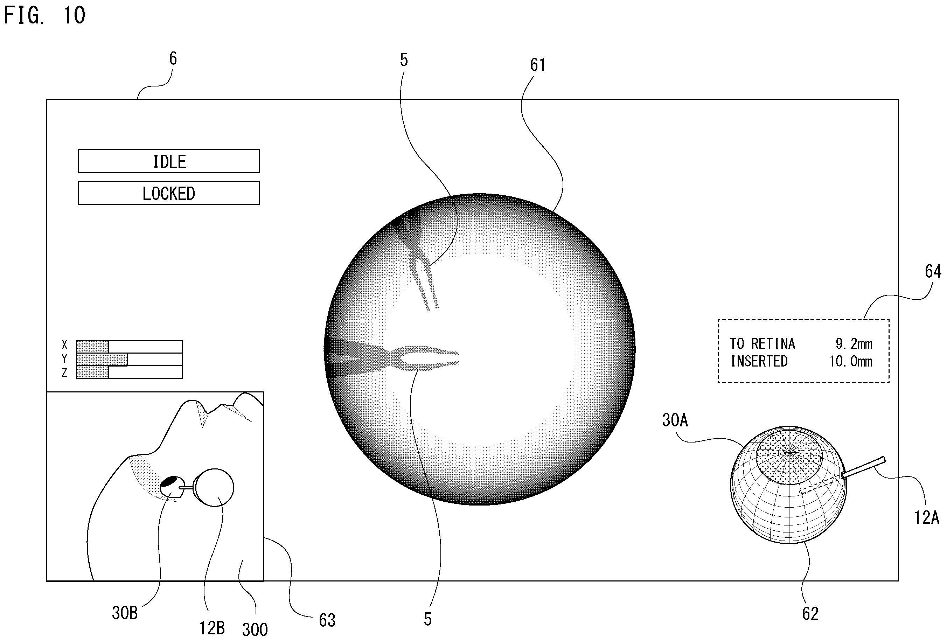

Incidentally, during the surgery on the eyeball 30 by the operator 4, treatment instruments 5 may appear in the endoscope captured image 61, as illustrated in FIG. 10. In this case, as is clear from the display positions of the treatment instruments 5, the upper and lower sides and left and right sides of the endoscope captured image 61 do not match a direction in which the operator 4 illustrated in FIG. 1 performs the surgery on the inside of the eyeball 30.

This is caused by the occurrence of a twist in the endoscope 12 about a longitudinal axis at a time of adjustment of the position of the endoscope 12 inserted into the eyeball 30. When the endoscope 12 images the inside of the eyeball 30 in a state in which such a twist has occurred, the endoscope captured image 61 whose upper and lower sides and left and right sides do not match the direction in which the operator 4 is performing the surgery is displayed on the monitor 15.

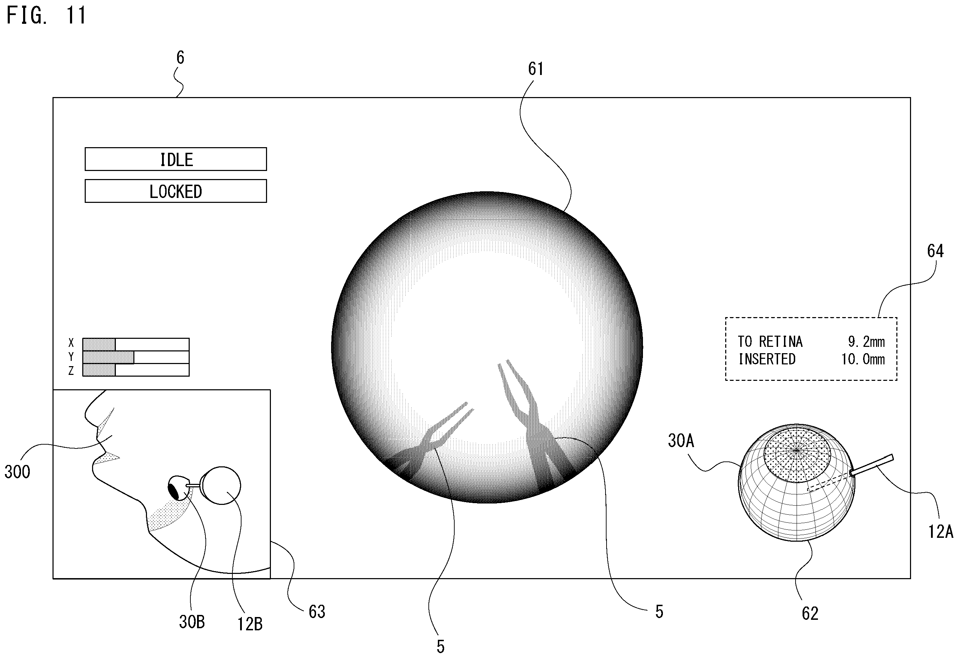

In such a case, the operator 4 needs to rotate the endoscope captured image 61 to an easily viewable position (for example, a position illustrated in FIG. 11) by operating the operating unit 13 in order to proceed with the surgery smoothly.

The computation and control unit 14 therefore performs the processing of steps S108 and S109 in FIG. 7.

In step S108, the computation and control unit 14 determines whether or not an operating input from the operating unit 13 is detected. An operating input is, for example, performed by the operator 4 by operating the foot pedal with a foot.

When no operating input is detected in step S 108, the computation and control unit 14 returns the processing to step S 102, and subsequently performs similar processing.

That is, in the initial state, the endoscope captured image 61 and the endoscope viewpoint map image 63 in a non-rotated state are displayed on the monitor 15.

When an operating input is detected in step S108, on the other hand, the computation and control unit 14 advances the processing from step S108 to S109.

In step S109, the computation and control unit 14 performs update processing on the image rotational angle data.

The computation and control unit 14 updates the rotational direction and the rotational angle as the image rotational angle data, on the basis of the operating input from the operating unit 13.

After performing the processing of step S109, the computation and control unit 14 returns the processing to step S102. Then, the computation and control unit 14 performs the processing from step S 102 to S 104, and thereby generates the image data of the endoscope captured image 61 obtained by rotating the captured image data from the endoscope 12 on the basis of the updated image rotational angle data.

When such processing is performed, the endoscope captured image 61 displayed on the monitor 15 can continue to be rotated according to the operation of the operating unit 13 without causing a positional displacement of the endoscope 12 within the eyeball 30. For example, the endoscope captured image 61 can be rotated from a position illustrated in FIG. 10 to a position illustrated in FIG. 11.

In the generation processing for the endoscope viewpoint map image 63 in the following step S105, the computation and control unit 14 generates the image data of the endoscope viewpoint map image 63 obtained by rotating the three-dimensional model image 300 on the basis of the updated image rotational angle data in a state in which the position of the endoscope model image 12B is fixed.

At this time, the three-dimensional model image 300 is rotated with respect to the fixed endoscope model image 12B. For example, the three-dimensional model image 300 is rotated from a position illustrated in FIG. 10 to a position illustrated in FIG. 11 with respect to the endoscope model image 12B in conjunction with the rotation of the endoscope captured image 61 from the position illustrated in FIG. 10 to the position illustrated in FIG. 11.

The image data of the endoscope viewpoint map image 63 rotated in conjunction with the rotation of the endoscope captured image 61 can be generated by thus rotating the three-dimensional model image 300 on the basis of the image rotational angle data.

Then, in step S106, the computation and control unit 14 generates the image data of the display image 6 including the rotated endoscope captured image 61 and the rotated endoscope viewpoint map image 63.

Then, in step S107, the computation and control unit 14 performs display control for displaying the display image 6 on the liquid crystal of the monitor 15.

As a result of the display control of the computation and control unit 14, the endoscope captured image 61 rotated according to the operation of the operating unit 13 by the operator 4 is displayed on the monitor 15. In addition, the endoscope viewpoint map image 63 rotated in conjunction with the rotation of the endoscope captured image 61 is displayed on the monitor 15.

When the endoscope captured image 61 is rotated, it may be difficult to spatially grasp the positional relation between the eyeball 30 and the endoscope 12 (for example, the inserted state (imaging direction) of the endoscope 12 with respect to the eyeball 30) from the video of the endoscope captured image 61. In such a case, it is possible to grasp the inserted state of the endoscope 12 with respect to the eyeball 30 easily by checking the endoscope viewpoint map image 63 rotated in conjunction with the rotation of the endoscope captured image 61.

While the operator 4 continues operating the operating unit 13, the computation and control unit 14 continues performing the processing from step S102 to step S109.

The operator 4, for example, continues operating the operating unit 13 to rotate the endoscope captured image 61 until the treatment instruments 5 appearing in the endoscope captured image 61 are located at positions that facilitate the surgery.

When the operator 4 ends the operation of the operating unit 13, the computation and control unit 14 determines that no operating input is detected in step S108. In this case, the computation and control unit 14 returns the processing from step S108 to S102, and thereafter repeatedly performs the processing from step S102 to step S108 until an operating input is detected in step S108.

At this time, the computation and control unit 14 generates the image data of the endoscope captured image 61 and the endoscope viewpoint map image 63 on the basis of the last updated image rotational angle data, and performs display control on the monitor 15. Thus, the endoscope captured image 61 and the endoscope viewpoint map image 63 are displayed on the monitor 15 in a state in which the rotational angle at a time point of a stop of the rotation is maintained.

Incidentally, the computation and control unit 14 does not update the image rotational angle data even when the endoscope 12 held by the holder 21 is moved by operation control on the arm unit 17 to shift the imaging direction of the endoscope 12.

Hence, on the monitor 15, the endoscope captured image 61 obtained by shifting only the imaging direction is displayed in a state in which the rotation based on the last updated image rotational angle data is maintained.

Hence, the operator 4 does not need to rotate the endoscope captured image 61 again by operating the operating unit 13 when the imaging direction of the endoscope 12 is shifted.

In addition, the endoscope viewpoint map image 63 maintains the state in conjunction with the rotational state of the endoscope captured image 61. Incidentally, when the imaging direction of the endoscope 12 is shifted, the position of the three-dimensional model image 300 with respect to the endoscope model image 12B in the endoscope viewpoint map image 63 is shifted.

When the computation and control unit 14 detects an operating input again in step S108 while repeatedly performing the processing from step S102 to step S108, the computation and control unit 14 advances the processing in order of steps S109 and S 102, and thereafter performs similar processing.

### <4. Summary>

The surgery assisting device 2 in the embodiment of the present invention includes the arm unit 17 including the holder 21 for holding the endoscope 12 and configured to adjust the position of the endoscope 12 in a state in which the holder 21 holds the endoscope 12, the image generating section 144 configured to generate the endoscope viewpoint map image 63 displaying the three-dimensional model image 300 of the subject 3 from the viewpoint of the endoscope 12 held by the holder 21, the display control section 145 configured to perform display control of the endoscope viewpoint map image 63 and the endoscope captured image 61 that is obtained by the endoscope 12, and the operating unit 13 for rotating the endoscope captured image 61. The displayed endoscope captured image 61 is rotated according to operation of the operating unit 13 without a positional displacement of the endoscope 12 being caused, and the endoscope viewpoint map image 63 is rotated in conjunction with the rotation of the endoscope captured image 61 (see FIG. 7, and FIG. 8, and the like).

The endoscope viewpoint map image 63 is consequently displayed which corresponds to the state of the endoscope captured image 61 after being rotated and indicates positional relation between the endoscope 12 and the subject 3.

Therefore, the operator 4 can spatially grasp the inserted state of the endoscope 12 with respect to the eyeball 30 of the subject 3. Hence, the operator 4 can perform surgery while intuitively grasping the positional relation even in a state in which the endoscope captured image 61 is rotated, and the operator 4 can thereby proceed with the surgery on the eyeball 30 smoothly. In addition, because the operator 4 can grasp the position of the endoscope 12, the operator 4 can avoid coming into contact with the endoscope 12 or the like.

In the surgery assisting device 2 in the present embodiment, the rotation of the endoscope viewpoint map image 63 is performed in a state in which the position of an image illustrating the endoscope 12 is fixed (see S105 in FIG. 7, S205 in FIG. 9, FIG. 10, FIG. 11, and the like).

Consequently, the endoscope viewpoint map image 63 is displayed in a state in which the image illustrating the endoscope 12 always appears irrespective of the rotational state of the endoscope captured image 61.

Therefore, by visually checking the endoscope viewpoint map image 63, it is possible to check the inserted state of the endoscope 12 with respect to the eyeball 30 of the subject 3 at all times irrespective of the rotation of the endoscope captured image 61. Hence, it is possible to proceed with the surgery on the eyeball 30 smoothly.

In the surgery assisting device 2 in the present embodiment, the display control section 145 displays the endoscope viewpoint map image 63 and the endoscope captured image 61 within the same screen (see FIG. 5, S107 in FIG. 7, and the like).

Consequently, in performing the surgery while visually checking the endoscope captured image 61, it is possible to check the endoscope viewpoint map image 63 without moving a line of sight to another monitor 15.

Hence, visibility of the endoscope viewpoint map image 63 is improved, and it is possible to proceed with the surgery on the eyeball 30 smoothly.

In the surgery assisting device 2 in the present embodiment, the display control section 145 displays the ocular map image 62 indicating the position of the endoscope 12 on a three-dimensional ocular model and the endoscope viewpoint map image 63 within the same screen (see FIG. 5, S107 in FIG. 7, and the like).

Consequently, in checking the endoscope viewpoint map image 63, it is possible to check the ocular map image 62 without moving the line of sight to another monitor 15.

Therefore, a plurality of pieces of information can be checked simultaneously on one monitor 15. It is thus possible to proceed with the surgery on the eyeball 30 smoothly.

It is to be noted that, while an example of an intraocular endoscope has been described as an example of the endoscope 12 in the present embodiment, the endoscope 12 is not limited to the intraocular endoscope. For example, it is possible to apply various endoscopes such as a thoracoscope inserted after an incision between ribs of the subject 3 and a laparoscope inserted after an incision in an abdomen.

Finally, the embodiment described in the present disclosure is merely illustrative, and the present invention is not limited to the foregoing embodiment but based on the scope of the invention as defined by the claims. In addition, all of combinations of the configurations described in the embodiment are not necessarily essential to solving the problems. Further, the effects described in the present disclosure are merely illustrative, and are not limited. Other effects may be produced, or a part of the effects described in the present disclosure may be produced.

### Reference Signs List

2: Surgery assisting device
3: Subject
12: Endoscope
13: Operating unit
17: Arm unit
21: Holder
30: Eyeball
30A: Three-dimensional ocular model image
30B: Three-dimensional ocular model image
61: Endoscope captured image
62: Ocular map image
63: Endoscope viewpoint map image
144: Image generating section
145: Display control section
300: Three-dimensional model image

## Claims

1. A surgery assisting device (2) comprising:
an arm unit (17) including a holder (21) for holding an endoscope (12) and configured to adjust a position of the endoscope (12) in a state in which the holder (21) holds the endoscope (12);
an image generating section (144) configured to generate an endoscope viewpoint map image (63) that is an image of a three-dimensional model of a subject from a viewpoint of the endoscope (12) held by the holder (21);
a display control section (145) configured to perform display control of the endoscope viewpoint map image (63) and an endoscope captured image (61) that is obtained by the endoscope (12); and
an operating unit (13) for rotating the endoscope captured image (61),
wherein a computation and control unit (14) is configured to rotate the displayed endoscope captured image (61) according to operation of the operating unit (13) without a positional displacement of the endoscope (12) being caused, and the computation and control unit (14) is further configured to rotate the endoscope viewpoint map image (63) in conjunction with the rotation of the endoscope captured image (61).

2. The surgery assisting device according to claim 1, wherein the computation and control unit is configured to perform the rotation of the endoscope viewpoint map image in a state in which a position of an image illustrating the endoscope is fixed.

3. The surgery assisting device according to claim 1 or 2, wherein the display control section is configured to display the endoscope viewpoint map image and the endoscope captured image within a same screen.

4. The surgery assisting device according to any one of claims 1 to 3, wherein the display control section is configured to display an ocular map image indicating the position of the endoscope on a three-dimensional ocular model and the endoscope viewpoint map image within a same screen.

## Patentansprüche

1. Chirurgiehilfsvorrichtung (2), enthaltend:
eine Armeinheit (17), die eine Halterung (21) zum Halten eines Endoskops (12) einschließt und konfiguriert ist, eine Position des Endoskops (12) in einem Zustand einzustellen, in dem die Halterung (21) das Endoskop (12) hält;
einen Bilderzeugungsteil (144), der konfiguriert ist, um ein Endoskopblickwinkelkartenbild (63) zu erzeugen, das ein Bild eines dreidimensionalen Modells eines Subjekts aus einem Blickwinkel des Endoskops (12) ist, das von der Halterung (21) gehalten wird;
einen Anzeigesteuerteil (145), der konfiguriert ist, eine Anzeigesteuerung des Endoskopblickwinkelkartenbildes (63) und eines Endoskop-erfassten Bildes (61) durchzuführen, das von dem Endoskop (12) erhalten wird; und
eine Bedieneinheit (13) zum Drehen des Endoskop-erfassten Bildes (61),
wobei eine Rechen- und Steuereinheit (14) konfiguriert ist, das angezeigte Endoskop-erfasste Bild (61) entsprechend einer Betätigung der Bedieneinheit (13) zu drehen, ohne dass eine Positionsverschiebung des Endoskops (12) verursacht wird, und die Rechen- und Steuereinheit (14) weiterhin konfiguriert ist, das Endoskopblickwinkelkartenbild (63) zusammen mit der Drehung des Endoskop-erfassten Bildes (61) zu drehen.

2. Chirurgiehilfsvorrichtung nach Anspruch 1, wobei
die Rechen- und Steuereinheit konfiguriert ist, die Drehung des Endoskopblickwinkelkartenbildes in einem Zustand durchzuführen, in dem eine Position eines Bildes festgesetzt ist, das das Endoskop veranschaulicht.

3. Chirurgiehilfsvorrichtung nach Anspruch 1 oder 2, wobei
der Anzeigesteuerteil konfiguriert ist, das Endoskopblickwinkelkartenbild (63) und das Endoskop-erfasste Bild auf demselben Bildschirm anzuzeigen.

4. Chirurgiehilfsvorrichtung nach einem der Ansprüche 1 bis 3, wobei
der Anzeigesteuerteil konfiguriert ist, ein Augenkartenbild, das die Position des Endoskops auf einem dreidimensionalen Augenmodell angibt, und das Endoskopblickwinkelkartenbild auf demselben Bildschirm anzuzeigen.

## Revendications

1. Un dispositif d'assistance chirurgicale (2) comprenant :
une unité de bras (17) comprenant un support (21) pour tenir un endoscope (12) et configurée pour ajuster une position de l'endoscope (12) dans un état dans lequel le support (21) tient l'endoscope (12) ;
une section génératrice d'images (144) configurée pour générer une image de carte de point de vue de l'endoscope (63) qui est une image d'un modèle tridimensionnel d'un sujet à partir d'un point de vue de l'endoscope tenu par le support (21) ;
une section de contrôle de l'affichage (145) configurée pour effectuer le contrôle de l'affichage de l'image de la carte du point de vue de l'endoscope (63) et d'une image capturée par l'endoscope (61) qui est obtenue par l'endoscope (12) ; et
une unité opératoire (13) pour tourner l'image capturée par l'endoscope (61),
dans lequel une unité de calcul et de contrôle (14) est configurée pour tourner l'image capturée par l'endoscope (61) affichée en fonction du fonctionnement de l'unité opératoire (13) sans qu'un déplacement positionnel de l'endoscope (12) ne soit causé, et l'unité de calcul et de contrôle (14) est configurée en outre pour tourner l'image de la carte du point de vue de l'endoscope (63) en conjonction avec la rotation de l'image capturée par l'endoscope (61).

2. Le dispositif d'assistance chirurgicale selon la revendication 1, dans lequel
l'unité de calcul et de contrôle (14) est configurée d'effectuer la rotation de l'image de la carte de point de vue de l'endoscope dans un état dans lequel une position d'une image illustrant l'endoscope est fixe.

3. Le dispositif d'assistance chirurgicale selon la revendication 1 ou 2, dans lequel
la section de contrôle de l'affichage est configurée pour afficher l'image de la carte du point de vue de l'endoscope et l'image capturée par l'endoscope sur un même écran.

4. Le dispositif d'assistance chirurgicale selon l'une quelconque des revendications 1 à 3, dans lequel
la section de contrôle de l'affichage est configurée pour afficher une image de carte oculaire indiquant la position de l'endoscope sur un modèle oculaire tridimensionnel et l'image de la carte du point de vue de l'endoscope sur un même écran.
